# EUROPEAN PATENT APPLICATION

(11) **EP 3 252 056 A1**
(43) Date of publication of application: **06.12.2017**
(21) Application number: 16171918.2
(22) Date of filing: 30.05.2016
(51) Int. Cl.: C07D 493/04

(54) **METHOD FOR PREPARATION OF MANNOSIDE DERIVATIVES**

(71) Applicant: Åbo Akademi University, 20500 Turku (FI)
(72) Inventor: Mavrynsky, Denys, 20500 Turku (FI); Leino, Reko, 20500 Turku (FI)
(74) Representative: Kolster Oy Ab

(57) **Abstract**

A method for preparation of a compound of formula (**I**) 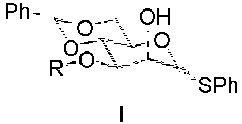 wherein R is H or *p*-methoxybenzyl (PMB) in which 1-thio-D-mannopyranoside is reacted with benzaldehyde dimethylacetal in acetonitrile in the presence of an acid catalyst at room temperature.

## Description

### FIELD OF THE INVENTION

The present invention relates to preparation of mannoside derivatives and more particularly to a synthetic method of preparing phenyl thiomannoside derivatives.

### BACKGROUND OF THE INVENTION

D. Crich et al. have disclosed (JACS 2004, 126, 15081-15086) a method for preparation of thioglycosides wherein phenyl 1-thio-*α*-D-mannopyranoside **(α-1)** is converted to a corresponding benzylidene acetal **(I**-**a**'**)** (and the corresponding *p*-methoxybenzylidene acetal) in what they call standard manner. Reaction with dibutyltin oxide (Bu₂SnO) and then *p*-methoxybenzyl chloride (PMBCl) and tetrabutylammonium bromide (Bu₄NBr) selectively afforded 3-*O*-PMB ether **(I-b')** in 94% yield.

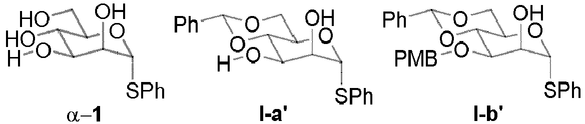

Product **(I**-**a**'**)** was first prepared by adding 1 equivalent of benzaldehyde dimethyl acetal to 1-thio-*α*-D-mannopyranoside **(α-1)** in DMF, in the presence of 10 mol% p-TsOH. The reaction mixture was then heated to 60 °C for 2 hours, then DMF was removed under vacuum and the reaction residue was diluted with a large amount of ethyl acetate (EtOAc) and washed with saturated aqueous NaHCO₃. The water phase was then extracted with EtOAc three times to prevent loss of product in the water phase, and the combined organic phase was washed with water and brine, dried over anhydrous MgSO₄, filtered and concentrated. The residue was crystallized from EtOAc to afford **(I**-**a**'**)** in 57% yield as a white solid.

Compound **(I-b')** was prepared by first adding to a solution of **(I**-**a**'**)** in toluene 1.02 equivalents of Bu₂SnO at room temperature (rt). After stirring the reaction mixture under reflux for 3 hrs, the reaction mixture was cooled down to rt and 1.06 equivalents of Bu₄NBr, 1.02 equivalents of CsF and 1.05 equivalents of PMBCl were added respectively. The reaction mixture was stirred under reflux for 3 hours before cooling down to rt. It was then poured into aqueous saturated Na-HCO₃, and extracted with EtOAc three times. The combined organic phase was washed with water and brine, dried over anhydrous MgSO₄, filtered through Celite, and concentrated. The crude reaction mixture was purified by column chromatography on silica gel (eluent: EtOAc/Hexane = 1/3 then 1/1) to give **(I-b')** in 94% yield as a white foam.

Fuwa et al. (Org. Lett. 2015, 17, 366-369) have disclosed a method for the preparation of *p*-methoxybenzylidene acetal of *p*-methoxyphenyl 1-thio-*α*-D-mannopyranoside **(2).** It is to be noted that the two extra CH₃O-groups present in the product **(2)** of the disclosed method significantly influence the solubility properties and crystallization patterns of said compound.

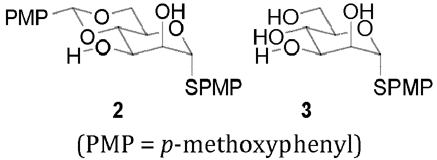

Compound **(2)** was prepared by adding to a solution of tetraol **(3)** in acetonitrile 1.5 equivalents of *p*-methoxybenzaldehyde dimethyl acetal and 20 mol% pyridinium *p*-toluenesulfonate (PPTS) at 0°C, and the resultant solution was stirred at 0 °C. The reaction was quenched with saturated aqueous NaHCO₃ solution at 0 °C. The resultant mixture was extracted with CH₂Cl₂, and the organic layer was washed with brine, dried over Na₂SO₄, filtered, and concentrated under reduced pressure. The residue was recrystallized from CH₂Cl₂/hexanes to give diol **(2)** in 52% yield as colorless crystals. To improve the overall yield to 75%, the mother liquor was concentrated under reduced pressure, and the residue was purified by flash column chromatography to give additional diol **(2)** in 23% yield as colorless crystals.

It can be observed from the presented ¹H NMR spectrum (on page S33 of the original article by Fuwa et al.) that the obtained product is not pure.

Problem of the existing synthetic procedures is that the preparation of the benzyl acetal **(I-a)** is often complicated by formation of a bis-addition by-product **(II).**

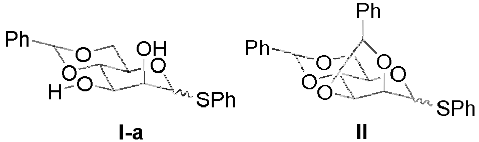

Both the target product **(I-a)** and the undesired by-product **(II)** have limited solubilities in most common organic solvents at room temperature. Solubility of the undesired by-product **(II)** at room temperature is notably lower compared to the target product **(I-a),** even in such typically efficient solvents as DMSO. Separation of the products by techniques other than chromatography, in particular crystallization, is thus challenging.

### BRIEF DESCRIPTION OF THE INVENTION

It is thus an object of the present invention to provide a method for the preparation of phenyl thiomannoside derivatives so as to alleviate the above disadvantages. The objects of the invention are achieved by a method which is characterized by what is stated in the independent claims. The preferred embodiments of the invention are disclosed in the dependent claims.

### BRIEF DESCRIPTION OF THE DRAWINGS

In the following the invention will be described in greater detail by means of preferred embodiments with reference to the attached drawings, in which
Figure 1 shows 1H NMR spectrum of the 1st crop of crystallization of **(I-a);** and
Figure 2 shows 1H NMR graph of different crops of **(I-a)** obtained by crystallization.

### DETAILED DESCRIPTION OF THE INVENTION

It has been surprisingly realized that particular synthetic conditions allow preparation and recovery of compound of formula **(I-a)** in the absence of compound of formula **(II).** Further it has been surprisingly realized that even when trace amounts of compound of formula **(II)** are formed during the synthetic procedure, pure compound of formula **(I-a)** can be recovered via particular recrystallization conditions. These realizations also allow preparation of compound of formula **(I-b)** in large scale and relatively low costs. 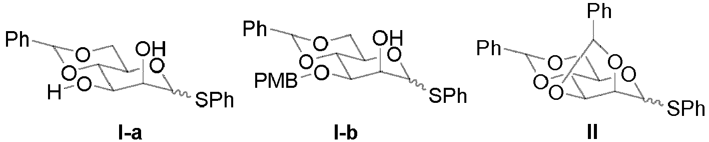 Accordingly provided herein is a method for preparation of a compound of formula **(I)** 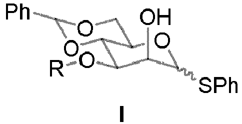 wherein R is H or *p*-methoxybenzyl (PMB),
comprising
(o) providing phenyl 1-thio-D-mannopyranoside;
(a) reacting 1 to 1.3 equivalents of benzaldehyde dimethylacetal (PhCH(OMe)₂) with 1 equivalent of phenyl 1-thio-D-mannopyranoside as a suspension in acetonitrile in the presence of 0.01 to 0.05 equivalents of an acid catalyst, such as toluene sulfonic acid (TSA), at room temperature to form compound of formula **(I-a)** as a precipitate, and recovering said precipitate by filtration 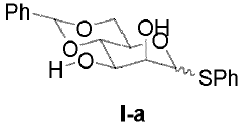 in the absence of a bis-addition by-product of formula **(II)** 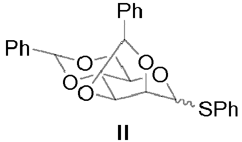
(b) suspending the precipitate obtained in step (a) in acetonitrile and recovering said precipitate by filtration;
(c) suspending the precipitate obtained in step (b) in *tert*-butyl methyl ether (TBME) and recovering said precipitate by filtration to obtain compound **(I-a);**
(d) optionally *p*-methoxybenzylating compound of formula **(I-a)** to obtain compound of formula **(I-b)**

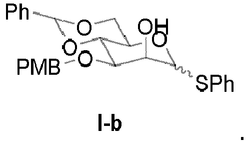

Preferably *p*-methoxybenzylation of compound of formula **(I-a)** in step (d) is accomplished by reacting an adduct of compound of formula **(I-a)** and Bu₂SnO with *p*-methoxybenzyl chloride (PMBCl). The said reaction is advantageously performed in the presence of a fluoride source and tetra-butyl ammonium halide (Bu₄NX, wherein X is halogen). Suitable fluoride sources include those selected from a group consisting of cesium fluoride (CsF), sodium fluoride (NaF), and tetra-butyl ammonium fluoride (Bu₄NF). Most preferably said reaction is performed in the presence of sodium fluoride (NaF) and tetra-butyl ammonium bromide (Bu₄NBr). The reaction could also be performed e.g. in the presence of tetra-butyl ammonium fluoride (Bu₄NF). In such case the tetra-butyl ammonium fluoride would also act as the fluoride source.

Further preferably compound of formula **(I-b)** is recovered by aqueous work-up.

Further provided herein are a method for preparation of a compound of formula **(I-b)** and/or a preferred method for accomplishing step (d) 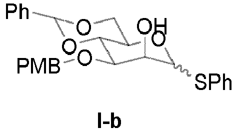 comprising
(e) reacting compound of formula **(I-a)** 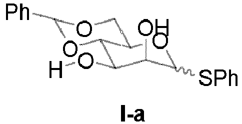 with a compound of formula R₂SnO, wherein each R is C₁₋₅-alkyl, preferably Bu₂SnO, to obtain as an intermediate product a compound of formula **(III)** 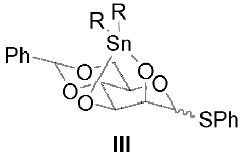 wherein each R is the same as in R₂SnO;
(f) reacting compound of formula **(III)** with *p*-methoxybenzyl chloride (PMBCl), preferably in the presence of a fluoride source and tetra-butyl ammonium halide (Bu₄NX, wherein X is halogen), to obtain compound of formula **(I-b)** 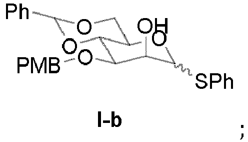 and
(g) optionally recrystallizing compound of formula **(I-b)** from a non-polar solvent, such as hexane.

The present method is particularly suitable for preparation of compound(s) of formula **(I)** in large scale, i.e. up to 10 kg scale, preferably for preparation of compound(s) of formula **(I)** in 100 g to 8 kg yield. This procedure is not as such practical for scale below 10 g. The present method avoids utilization of laborious chromatographical purification such as column chromatography and therefore significantly reduces production costs of compounds of formula **(I).** Also the costs of the required reagents are minimized.

Preferably step (a) is accomplished by adding benzaldehyde dimethylacetal (PhCH(OMe)₂) to a suspension of phenyl 1-thio-*α*-D-mannopyranoside in acetonitrile in presence of an acid catalyst at room temperature, and stirring the reaction mixture at rt.

The acid catalyst used in step (a) is typically an organic sulfonic acid, preferably selected from a group consisting of toluene sulfonic acid (TSA) and camphor sulfonic acid (CSA). Most preferably said acid catalyst is toluene sulfonic acid (TSA).

Use of benzaldehyde dimethylacetal (PhCH(OMe)₂) as the alkylating agent and acetonitrile as the solvent as well as avoiding an aqueous work up of the product, are essential in step (a) for obtaining compound **(I-a)** in the absence of a bis-addition by-product of formula **(II).**

Although compound of formula **(I-a)** obtained by the present method is free from the undesired bis-addition by-product of formula **(II),** the product may still contain low amounts of other unidentified minor impurities. If desired, compound **(I-a)** obtained in step (c) may be further purified by recrystallization. Accordingly the present method may further comprise:
(i) purifying the compound of formula **(I-a)** obtained from step (c) by dissolving the precipitate obtained in step (c) in a recrystallization solvent selected from a group consisting of ethanol, ethyl acetate and mixtures thereof, at boiling temperature of said solvent and allowing the thus obtained solution to cool down to RT to precipitate compound of formula **(I-a);** and filtering the precipitate to recover pure compound of formula **(I-a).** Preferably ethanol or ethyl acetate is used as the recrystallization solvent in the precipitation step (i).

Also, if it is desirable to improve the overall yield of compound of formula **(I-a),** the filtrate obtained in step (c) may be concentrated to a reduced volume, preferably to 40 to 60% of the original volume, more preferably to a volume whereby the filtrate has MeOH:acetonitrile ratio below 1:30, to form a second crop of precipitate comprising compound of formula **(I-a).** However, this is not recommended as this second crop may also comprise traces of said undesired bis-addition by-product **(II).** The impurities of the direct product of step (c) and/or the undesired bis-addition by-product **(II)** and possible other impurities of the second crop may be removed by subjecting the respective precipitates to recrystallization.

For improving the overall yield of compound of formula **(I-a),** the present method advantageously further comprises:
(ii) concentrating the filtrate obtained from filtration of step (c) to a reduced volume (as discussed above) to form a precipitate comprising compound of formula **(I-a)** and filtering, and optionally drying, the said precipitate to recover compound of formula**(I-a);** and
(iii) dissolving the precipitate obtained in step (ii), optionally together with the precipitated obtained in step (c), in a recrystallization solvent selected from a group consisting of ethanol, ethyl acetate and mixtures thereof, at boiling temperature of said solvent and allowing the thus obtained solution to cool down to RT to precipitate compound of formula **(I-a);** and filtering the precipitate to recover pure compound of formula **(I-a).** Preferably ethanol or ethyl acetate is used as the recrystallization solvent in the precipitation step (iii).

Most preferably the recrystallization solvent used in step (i) and/or in step (iii) is ethanol (EtOH), in particular 96% EtOH, as the use of ethanol provides high yield with high purity i.e. no traces of impurities detectable by ¹H NMR, even when used as an azeotrope with water i.e. as 96% EtOH.

Preferably the solvent used in step (e) is toluene. Said toluene does not require being pre-dried, both dry and wet toluene are suitable.

Further preferably step (e) is accomplished by dissolving and reacting compound of formula **(I-a)** and R₂SnO in toluene by bringing the reaction mixture into reflux.

As already discussed above, preferably step (f) is accomplished by reacting **(III)** with *p*-methoxybenzyl chloride (PMBCl). The said reaction is advantageously performed in the presence of a fluoride source and tetra-butyl ammonium halide (Bu₄NX, wherein X is halogen). Suitable fluoride sources include those selected from a group consisting of cesium fluoride (CsF), sodium fluoride (NaF), and tetra-butyl ammonium fluoride (Bu₄NF). Most preferably said reaction is performed in the presence of sodium fluoride (NaF) and tetra-butyl ammonium bromide (Bu₄NBr). The reaction could also be performed e.g. in the presence of tetra-butyl ammonium fluoride (Bu₄NF). In such case the tetra-butyl ammonium fluoride would also act as the fluoride source. Use of expensive cesium salt in step (f) is not necessary in water-containing media. In step (f) CsF is successfully replaced by use of the significantly cheaper NaF. Test run on 10 mmol scale showed no difference compared to CsF.

Step (f) is preferably accomplished by cooling the reaction mixture until boiling has ceased before adding PMBCl, tetra-butyl ammonium halide and the fluoride source.

When a compound of formula **(I-b)** is purified by recrystallization in step (g), it is preferably accomplished by extracting the compound of formula **(I-b)** with a non-polar solvent, such as hexane, as the solvent in a Soxhlet extractor and allowing the purified compound of formula **(I-b)** to precipitate in the solvent flask of the Soxhlet extractor.

The non-polar solvent used in step (g) is preferably selected from C₆₋₈-alkanes, in particular from a group consisting of hexane, heptane, isooctane and mixtures thereof. Most preferably the non-polar solvent is hexane.

Before recrystallization of the compound of formula **(I-b)** from a non-polar solvent, such as hexane, the crude compound is preferably dissolved in a hydrophilic solvent, such as tert-butyl methyl ether (TBME), in the presence of celite and the hydrophilic solvent is then evaporated. The non-polar solvent is preferably a di (C₁₋₆-alkyl)ether, such as tert-butyl methyl ether (TBME), or C₁₋₆-alkylacetate, such as ethyl acetate (EtOAc). Preferably the non-polar solvent is tert-butyl methyl ether (TBME).

The obtained solid may then be re-evaporated with the non-polar solvent, such as hexane. Formation of the solid mixture of the crude compound **(I-b)** and celite renders loading of the crude compound **(I-b)** into Soxhlet extractor easier. The obtained solid is then grinded and recrystallized from hexane in a Soxhlet extractor as described above.

Minor amounts of anisyl alcohol originating from hydrolysis of PMBCl may be present in the compound of formula **(I-b).** If desired, this impurity may be removed by crystallization from TBME-hexane.

When compound of formula **(I-b)** is obtained as a mixture of anomers the isomers can be separated, if desired, by crystallization based on lower solubility of the β-isomer in dichloromethane/hexane mixture. Crystallization from TBME-hexane does not influence the ratio of the anomers.

In accordance with the present method phenyl 1-thio-mannopyranoside **(1)** is preferably provided in step (o) by hydrolyzing tetraacetate of formula **(10)** 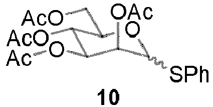 in methanol (MeOH) in the presence of a catalytic amount of sodium methoxide (MeONa) and neutralizing the obtained reaction mixture with concentrated aqueous HCl and methanol, evaporating the obtained reaction mixture; adding acetonitrile to the obtained residue; and re-evaporating the obtained solution to recover phenyl 1-thio-D-mannopyranoside **(1)**

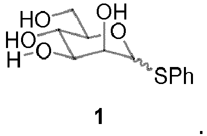

The use of HCl allows to avoid filtration steps while the formed NaCl is inert and will not affect further transformation steps and will eventually be removed by water work-up during the subsequent synthetic steps. By following the above procedure phenyl 1-thio-D-mannopyranoside **(1)** is obtained as an anomeric mixture enriched in the α-form **(*α*-1).**

### EXAMPLES

### Example 1: Preparation of compound (1)

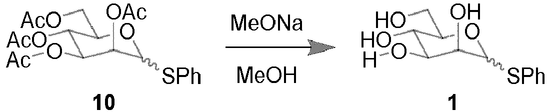

Tetraacetate **(10)** was hydrolysed in methanol (MeOH) in the presence of 2.5 mol% of sodium methoxide (MeONa). The reaction mixture was neutralized with a mixture of 3 mL of concentrated aqueous HCl and 40 mL of MeOH. The obtained mixture was evaporated and then re-evaporated with acetonitrile (AN). Compound **(1)** was obtained as a viscous mass as an anomeric mixture α/β in approx. 10:1 ratio.

### Example 2: Preparation of compound (I-a)

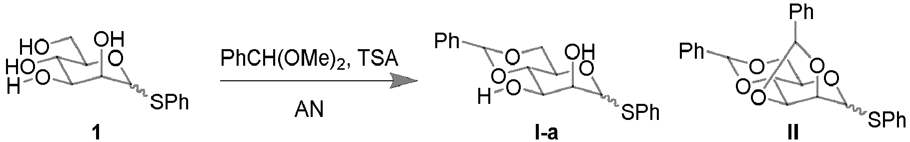

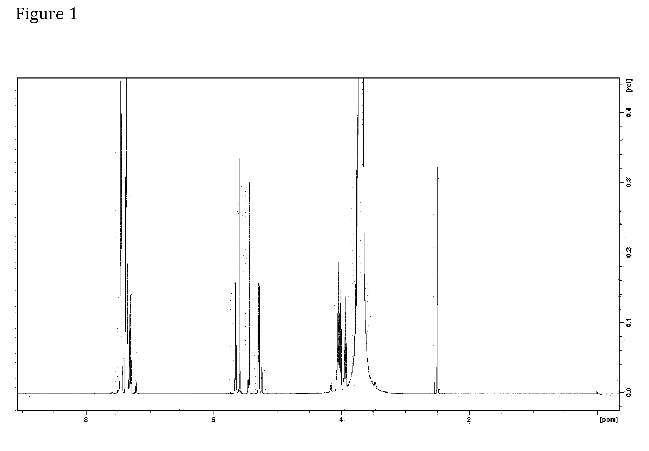

233 g of **(1)** (0.79 mol) was suspended in 3L of acetonitrile (AN) using mechanical stirrer at 300 rpm. 5 g (26 mmol, 3 mol%) of toluene sulfonic acid hydrate (TSA*H₂O) was added. Then 150 mL (1 mol) of benzaldehyde dimethylacetal (PhCH(OMe)₂) was added dropwise during 2 h at RT. The reaction mixture turned homogeneous after the first 20 ml of PhCH(OMe)₂ had been added. The reaction mixture was stirred at rt overnight. The formed white thick precipitate was filtered off, re-suspended in 0.5 L of acetonitrile, filtered off, re-suspended in 0.5 L of tert-butyl methyl ether (TBME), filtered off, air dried, grinded in a mortar and vacuum-dried (rotary evaporator, 10 mbar, 40 °C water bath). As a first crop 233 g (82%) of compound **(I-a)** was obtained. According to 1H NMR no bis-product **(II)** was detected in this batch as can be seen from the NMR spectra of Figure 1.

The mother liquor from the filtration was concentrated to approximately half of the original volume. The formed precipitate was filtered off, washed on filter with acetonitrile (2 x 100 mL), dried in air, grinded in a mortar and dried in vacuum. 19.6 g (additional 7%) of compound **(I-a)** was obtained as the second crop. According to 1H NMR the product contained traces of undesired bis-product **(II).**

### Example 3: Recrystallization of compound (I-a)

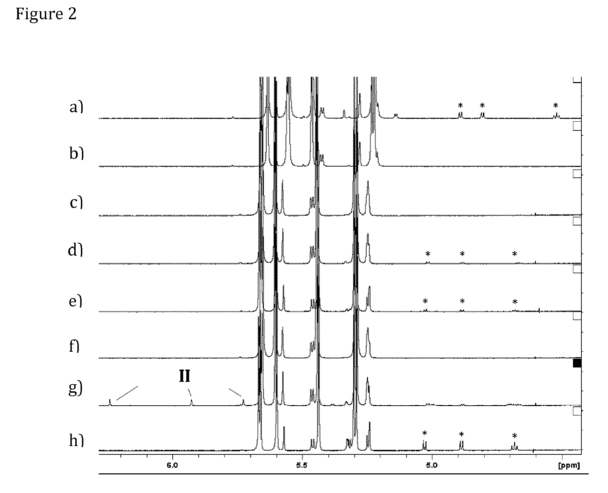

Crude compound **(I-a)** from the 1^{st} crop of Example 2 was dissolved in a solvent of Table 1 (17 - 25 g/L) at boiling point of the said solvent and left overnight to cool down to rt. EtOH and EtOAc allow higher concentration and therefore are consumed in less quantities as compared to methanol. The precipitate was filtered off on glass filter, washed with respective cold solvent and dried first in air, then on rotary evaporator. Purity of the obtained precipitates was evaluated with 1H NMR, as shown in Figure 2. In Figure 2, a) is 2^{nd} crop from EtOH with large loading, b) is 1^{st} crop from EtOH with large loading, c) is 1^{st} crop from EtOH, d) is 1^{st} crop from EtOAc, e) is 2^{nd} crop from MeOH, f) is 1^{st} crop from MeOH, g) is 2^{nd} crop from reaction mixture with acetonitrile (procedure as in Example 2), and h) is 1^{st} crop from reaction mixture with acetonitrile (procedure as in Example 2).

**Table 1. Recrystallization of I-a in different solvents**

| Solvent | Dissolved precipitate | Amount of solvent | Yield | Purity |
|---|---|---|---|---|
| MeOH | 23 g | 1.3 L | 12.7g (55%) | Pure |
| EtOAc | 30 g | 1.3 L | 23.7 g (79%) | Minor impurities |
| 96% EtOH | 31g | 1.1 L | 24.5 g (79%) | Pure |
| 96% EtOH | 170g | 6.9 L | 135 g (79%) | Pure |

### Example 4: Preparation of compound (I-b)

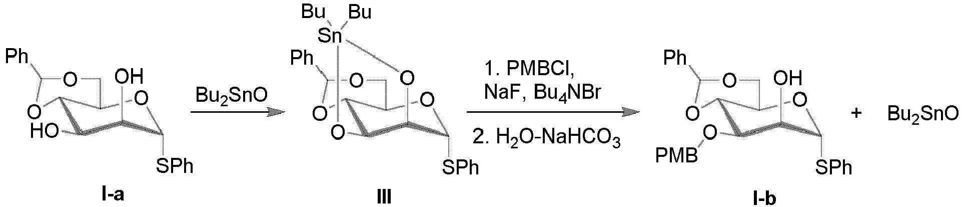

First, 90 g (0.25 mol) of **(I-a),** 63.7 g (0.26 mol) of Bu₂SnO and 0.8 L of toluene (from flask, not pre-dried) were introduced to 2 L one-necked roundbottom flask equipped with a magnetic stirrer and reflux condenser and refluxed for 30 min without protection from air atmosphere. After this time most of the solid material was dissolved and the reaction mixture was transparent. The reaction mixture was then cooled down until boiling had ceased whereafter 11.5 g (0.275 mol) of NaF, 88.5 g (0.275 mol) of Bu₄NBr and 39 mL (0.25 mol) of PMBCl were added. PMBCl was prepared by the standard procedure from anisyl alcohol and conc. HCl and re-evaporated with toluene. The obtained product displayed 1.11 g/ml density and contained 10.5 mass% of toluene (according to ¹H NMR). Boiling was then continued for 2.5 hours. Monitoring of the reaction by TLC demonstrated that already after 1.5 hours there is no further progress of the reaction. The reaction mixture was cooled down to RT and concentrated in rotary evaporator. The residue was mixed with 1 L of TBME (insoluble) and 0.5 L of saturated aqueous NaHCO₃ and stirred with a mechanical stirrer for 2 hrs. The precipitated Bu₂SnO was filtered off by using a 3 cm thick pad of coarse celite (Celite 535 from Sigma-Aldrich) on a Schott glass filter (13 cm diameter, porosity #2). The filtered precipitate was collected, boiled with 0.5 L of TBME and filtered through celite again. The washing procedure was repeated three more times. The filtrates were combined, the organic phase was separated, washed with water, brine, dried over Na₂SO₄ and concentrated using a rotary evaporator, providing 140 g of a viscous material crystallizing upon storage. Mass content of MTBE - 18% (according to ¹H NMR), product content - approx. 115 g (96%).

The crude product was dissolved in 0.6 L of TBME, mixed with 55 g (200 mL) of coarse celite, evaporated in rotary evaporator and re-evaporated with hexane. The obtained solid was grinded in a mortar and loaded into 450 mL Soxhlet extractor. The solvent flask (2 L) was filled with 1.5 L of hexane. The extraction was continued for 42 h with extraction cycle duration 7-10 min. The extracted product crystallizes in the solvent flask already at boiling temperature forming large crystals. The extract was cooled down to RT and then to +4 °C. Hexane was poured away and the solid leftovers were thoroughly washed with hexane at room temperature. The evaporated hexane fractions did not contain product (according to ¹H NMR) and were discarded. Crystals were scratched from flask walls, grinded and dried in rotary evaporator providing 104.2 g (87%) of the product. Leftovers stuck on the flask walls were dissolved in TBME and concentrated, providing additional 4.7 g (85 mass % assay, 4 g, 3%) of the product **(I-b).**

It will be obvious to a person skilled in the art that, as the technology advances, the inventive concept can be implemented in various ways. The invention and its embodiments are not limited to the examples described above but may vary within the scope of the claims.

## Claims

1. A method for preparation of a compound of formula **(I)** 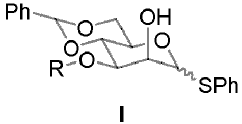 wherein R is H or *p*-methoxybenzyl (PMB),
comprising
(o) providing phenyl 1-thio-D-mannopyranoside;
(a) adding 1 to 1.3 equivalents of benzaldehyde dimethylacetal (PhCH(OMe)₂) to a suspension of 1 equivalent of phenyl 1-thio-*α*-D-mannopyranoside in acetonitrile in the presence of 0.01 to 0.05 equivalents of an acid catalyst, such as toluene sulfonic acid (TSA), at room temperature (rt), stirring the reaction mixture at rt to form compound of formula **(I-a)** as a precipitate, and recovering said precipitate by filtration; 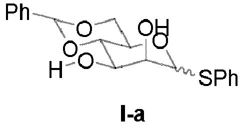 in the absence of a bis-addition by-product of formula **(II)** 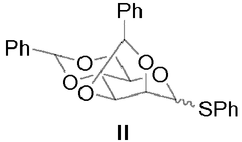
(b) suspending the precipitate obtained in step (a) in acetonitrile and recovering said precipitate by filtration;
(c) suspending the precipitate obtained in step (b) in *tert*-butyl methyl ether (TBME) and recovering said precipitate by filtration to obtain compound **(I-a);**
(d) optionally *p*-methoxybenzylating compound of formula **(I-a)** to obtain compound of formula **(I-b)**

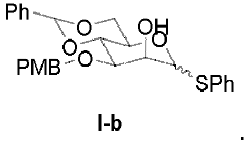

2. A method as claimed in claim 1, wherein step (d) is accomplished by reacting compound of formula **(I-a)** with *p*-methoxybenzyl chloride (PMBCl).

3. A method as claimed in claim 2, wherein step (d) is accomplished in the presence of sodium fluoride (NaF) and tetra-butyl ammonium bromide (Bu₄NBr).

4. A method as claimed in any one of claims 1 to 3, wherein the method further comprises:
(i) purifying the compound of formula **(I-a)** obtained from step (c) by dissolving the precipitate obtained in step (c) in a solvent selected from a group consisting of ethanol, ethyl acetate and mixtures thereof, at boiling temperature of said solvent and allowing the thus obtained solution to cool down to RT to precipitate compound of formula **(I-a);** and filtering the precipitate to recover pure compound of formula **(I-a).**

5. A method as claimed in any one of claims 1 to 4, wherein the filtrate obtained in step (c) is concentrated to a reduced volume, preferably to 40 to 60% of original volume, more preferably to a volume whereby the filtrate has MeOH:acetonitrile ratio below 1:30 to form a precipitate comprising compound of formula **(I-a).**

6. A method as claimed in any one of claims 1 to 4, wherein the method further comprises:
(ii) concentrating the filtrate obtained from filtration of step (c) to a reduced volume, preferably to 40 to 60% of original volume, more preferably to a volume whereby the filtrate has MeOH:acetonitrile ratio below 1:30, to form a precipitate comprising compound of formula **(I-a)** and filtering and optionally drying the said precipitate to recover compound of formula **(I-a);** and
(iii) dissolving the precipitate obtained in step (ii), optionally together with the precipitated obtained in step (c), in a solvent selected from a group consisting of ethanol, ethyl acetate, and mixtures thereof, at boiling temperature of said solvent and allowing the thus obtained solution to cool down to rt to precipitate compound of formula **(I-a);** and filtering the precipitate to recover pure compound of formula **(I-a).**

7. A method as claimed in claim 4 or 6, wherein the solvent used in step (i) and/or in step (iii) is ethanol (EtOH), in particular 96% EtOH

8. A method as claimed in any one of claims 1 to 7, wherein phenyl 1-thio-mannopyranoside **(1)** is provided in step (o) by hydrolyzing tetraacetate of formula **(10)** 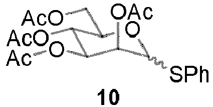 in methanol (MeOH) in the presence of a catalytic amount of sodium methoxide (MeONa) and neutralizing the obtained reaction mixture with concentrated aqueous HCl and methanol, evaporating the obtained reaction mixture; adding acetonitrile to the obtained residue; and re-evaporating the obtained solution to recover phenyl 1-thio-D-mannopyranoside **(1)**

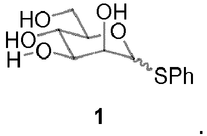

9. A method as claimed in any one of claims 1 to 8, wherein the yield of compound of formula **(I)** is from 100 g to 8 kg.

10. A method as claimed in any one of claims 1 to 9 wherein step (d) is accomplished by
(e) reacting compound of formula **(I-a)** 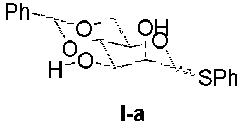 with a compound of formula R₂SnO, wherein each R is C₁₋₅-alkyl, preferably Bu₂SnO, to obtain as an intermediate product a compound of formula **(III)** 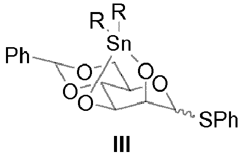 wherein each R is C₁₋₅-alkyl;
(f) reacting compound of formula **(III)** with PMBCl to obtain compound of formula **(I-b)** 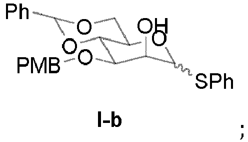 and
(g) optionally recrystallizing compound of formula (I-b) from a non-polar solvent.

11. A method as claimed in claim 10, wherein the non-polar solvent is hexane.

12. A method for preparation of a compound of formula **(I-b)** 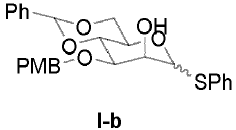 comprising
(e) reacting compound of formula **(I-a)** 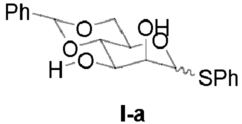 with a compound of formula R₂SnO, wherein each R is C₁₋₅-alkyl, preferably Bu₂SnO, to obtain as an intermediate product a compound of formula **(III)** 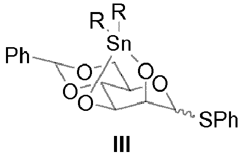 wherein each R is the same as in R₂SnO;
(f) reacting compound of formula **(III)** with PMBCl to obtain compound of formula **(I-b)** 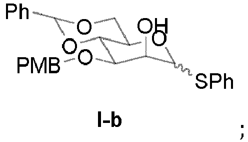 and
(g) optionally recrystallizing compound of formula **(I-b)** from a non-polar solvent, such as hexane.

13. A method as claimed in any one of claims 10 to 12, wherein the solvent used in step (e) is toluene.

14. A method as claimed in any one of claims 10 to 13, wherein R₂SnO is Bu₂SnO and in compound of formula **(III)** each R is butyl.

15. A method as claimed in any one of claims 10 to 14, wherein step (g) is accomplished by extracting the compound of formula **(I-b)** in the presence of hexane as the solvent in a Soxhlet extractor and allowing the purified compound of formula **(I-b)** to precipitate in the solvent flask of the Soxhlet extractor.
